# EUROPEAN PATENT SPECIFICATION

(11) **EP 1 366 716 B1**
(45) Date of publication and mention of the grant of the patent: **27.04.2005**
(21) Application number: 01948119.1
(22) Date of filing: 21.06.2001
(51) Int. Cl.: A61B 17/00

(54) **INTRAGASTRIC BALLOON ASSEMBLY**
INTRAGASTRISCHE BALLONANORDNUNG
ENSEMBLE BALLON INTRAGASTRIQUE

(30) Priority: 09.03.2001 MX 0000069
(43) Date of publication of application: 03.12.2003
(73) Proprietor: Garza Alvarez, José Rafael, México, D.F. 03100 (MX)
(72) Inventor: Garza Alvarez, José Rafael, México, D.F. 03100 (MX)
(74) Representative: Roshardt, Werner Alfred, Dipl.-Phys.
(86) International application number: PCT/MX2001/000039
(87) International publication number: WO 2002/071951

(56) References cited:
- EP-A1- 0 137 878
- EP-A1- 0 246 999
- US-A- 4 416 267
- US-A- 4 723 547
- US-A- 4 739 758
- US-A- 4 899 747

## Description

### Field of the Invention

The present invention refers to a kind of medical devices used to reduce the food intake of persons suffering from overweight problems, specifically persons with morbid obesity.

### Backgrounds of the Invention

The exogenous obesity, also named as morbid obesity or other names, generally means a weight increase in a significant proportion according to height, age, etc., and that may be risky for life due to the problems caused by the overweight itself. Before this medical circumstance, diverse medical treatments for weight reduction generally fail, therefore, surgical procedures have been the only possibility of treatment with an actual effectiveness, in most cases.

The obesity problem is so significant in developed countries that it has been considered as a public health problem, so the most important objective of any therapeutic is trying to solve this ailment with more effective treatments involving less complications, morbi-mortality and cost.

During the sixties and seventies, surgical experiences trying to reduce overweight were made and reported, by creating derivations that produce a short intestine effect, usually having disastrous results, since morbi-mortality was so high that caused almost a total abandonment thereof, until surgical devices appeared such as staplers, that arrived to our country at the end of the seventies, early eighties, starting a series of works published throughout the world, mainly about gastroplasties and derivations with a plurality of variations, results of which were good as to weight control and decrease refer, but open surgery was needed, having therefore risks in surgery, anesthesia, etc., in addition to high cost.

More recently, about 5 years ago, a new surgical technique appeared, consisting in placing by laparoscopy, an inflatable band encircling the stomach on its upper part, by means of a subcutaneous valve that may be controlled by radiology and/or endoscopy. Through this technique, a gastric reduction and food intake decrease are produced, resulting in a significant weight loss, practically the same as the one obtained by open surgery with staplers, but with less morbi-mortality and the advantages of the laparoscopic surgery, i.e., less pain and fast postoperative recovery. However, the most important problem of this surgical technique is the cost increase, as it is also an hospital, surgical and laparoscopic procedure, in addition to the band cost and specialized and experienced surgeons requirement.

At early eighties, the first reports on the use of intragastric balloons for obesity treatment appeared, which partially fill the stomach to produce a feeling of satiety, thus helping to reduce the food intake and to adopt new eating habits, such as the balloon disclosed in European Patent No. 137 878 of Lloyd R. Garren et al. consisting in a flexible inflatable balloon which in use is free-floating and unattached. Since then and during the following 10 years, approximately, publications of scientific literature of several countries have arisen about diverse experiences on the use of balloons made of different materials. Most of those balloons reported failures or very low success percentages, due to many complications and troubles during the placing procedure, handling and surveillance within the treatment period and therefore, the method lost credibility and in many cases it was abandoned, except for isolated experiences, reported in diverse publications. Currently, Bioenterics Corporation manufactures and distributes an intragastric balloon named BIB® System, which is seldom used, because the said company recommends it only as an alternative for limited reduction short programs or in some cases, prior to a definitive surgery, for which it is recommended a gastric capacity reduction surgery via lamparascopy by applying a gastric band, produced by such company.

The most significant problems arising from the use of intragastric balloons were, on the one hand, the manufacturing material, as in the beginning, elements that were easily destroyed by gastric juices were used, so the balloon should be frequently replaced, however, nowadays, this issue has been solved by the use of a resistant silicone material that is able to stay on several months inside the patient. Other important difficulties are the balloon rupture which consequently causes its migration to the intestine, eventually causing an intestine obstruction requiring surgery, and also the inflated balloon migration causing an esophagus or pylorus obstruction. Even more, the balloons available at the market show a great technical difficulty for endoscopic placing and the inflating maintenance or modification once it has been placed in the stomach.

There are several documents describing intragastric balloons with improvements intended to overcome some of these inconveniences including the one described in the US Patent No. 5,259,399 of Alan Brown, consisting in a balloon that is placed on the stomach fundus by means of a percutaneous gastrostomy and it is fixed up to the patient's skin, with a rigid or semi-rigid thick device, introducing the balloon there through which is then inflated and deflated with solutions and controlled by sensors placed on the patient's body and a battery driven electric pump, placed out of the patient's body. This device has the disadvantage that the patient must suffer a probe or thick catheter out of the abdomen skin and such probe or catheter has to be connected to the inflating or deflating equipment each time any food is ingested, resulting very uncomfortable for the patient.

On the other hand, US Patent No. 5,234,454 of Roger G. Bangs describes an intragastric balloon placed through a surgical gastrostomy previously made (several weeks before), to place the balloon later, implying a prior surgery. In addition, the used probe is very thick, approximately 1 cm diameter or more, and it stays externally which means that the patient must have a probe out of his/her body through a puncture made on the abdominal wall which, in addition to the inconveniences found in the Alan Brown US Patent, causes constant pain and discomfort to the patient. Bang's device has two balloons, one to be inflated and to maintain the stomach placed against the abdominal wall on the inside and a distal balloon used to fill the stomach.

The European Patent No. 0 137 878 (Mary L. and Lloyd R. Garren) relates to another stomach insert for treating obesity. The stomach volume is again reduced by a flexible, inflatable balloon. The balloon is free floating and unattached. At least a portion of the balloon features a self-sealing substance for sealing a puncture made for inflating the balloon. The insert is used by introducing a stomach tube into the stomach, the tube containing the balloon and an insufflation tube releasably attached to the balloon. However, again the free floating balloon may cause an esophagus or pylorus obstruction.

Therefore, there is a need for a means to treat the morbid obesity, allowing to reduce the surgical and postoperatory hazards which also substantially eliminates the patient's discomfort and decreases the treatment costs.

### Summary of the Invention

The object of the present invention is therefore, to provide an intragastric balloon assembly that remains fixed, avoiding the balloon migration and the possibility of digestive tube obstruction or blockade.

Another object of the invention is to cause the patient less anesthesic-surgical trauma to the patient during the intragastric balloon implantation.

It is also an object of the invention to provide the elements to control the size of the inflated intragastric balloon at any time after placement.

Even more, the balloon assembly of the invention intends both to avoid the discomfort caused by external elements and to make less evident the presence of the assembly's elements on the patient's body.

Likewise, by the present invention it is intended to decrease the hospital costs, by making a short stay or ambulatory surgery, and notwithstanding that the balloon and other assembly elements may have similar or lesser costs than current medical devices, the high cost of a laparoscopic surgery will be eliminated.

In addition, during the placing of the intragastric balloon of this invention, minimum personnel will be required, such as endoscopist, a surgeon, an assistant, a surgical nurse and anesthesia staff, reducing likewise the total cost.

Three years ago, a procedure to carry out no open surgery gastrostomies was started as a surgical endoscopic procedure, thus making easier the implantation of the gastrostomy probe by percutaneous puncture, previously inflating the stomach and surveying it with a panendoscope. This procedure may be carried out with local sedation and anesthesia, with less anesthetic-surgical trauma, thinking thus in an efficient option for the implantation of the intragastric balloon.

In order to comply with these objectives, the present invention combines the three medical-surgical elements above-described, such as an intragastric balloon, a valve for post-operatory inflating control, and the percutaneous gastrostomy technique with endoscopic control, as well as a new mean for the assembly traction and fixing, avoiding the balloon migration.

Therefore, a silicone balloon has been designed, having a fixed inflated catheter and an attachment consisting in a firmer silicone bar at the entrance of the balloon, to be used as tension support and to fix it to the aponeurosis, by means of a plate adjustable according to the needs and that may be fixed with suture points or metallic staples to the aponeurosis. Upon fixing these elements, the support is cut and the catheter is connected to an inflating valve, which is subcutaneously placed and attached to the aponeurosis through points or staples.

This way, elements external to the abdomen skin are avoided, such as probes or thick trocars, uncomfortable for the patient and that are difficult to control, as the invention assembly remains fixed to the abdominal aponeurosis and the inflating and deflating valve remains subcutaneously. Likewise, the use of other elements such as an additional balloon is avoided, as the air inflated balloon itself is used to make traction and place the stomach against the abdominal wall, and this way, it is allowed to reduce the diameter of the catheter or the inflating and fixing system.

It is important to note that by this technique, post operatory annoyances are minimized and the only drugs required are antibiotics as prophylaxis, and pain-relievers to control any discomfort. Surveillance is taken at the doctor's office once a month and a radiological control to verify and modify the balloon inflating will take place only in special occasions.

### Brief Description of Drawings.

Figure 1 shows the intragastric balloon assembly of the present invention;
Figure 2 shows an embodiment of the intragastric balloon assembly of the present invention;
Figure 3 shows a flat upper view of the fixing plate of the present invention;
Figure 4 shows a flat lateral view of the fixing plate of the present invention;
Figures 5 to 10 are schemes showing the surgical technique to implant the intragastric balloon assembly of this invention;
Figure 11 is a scheme showing how the intragastric balloon assembly of this invention is implanted.

### Detailed Description of the Invention

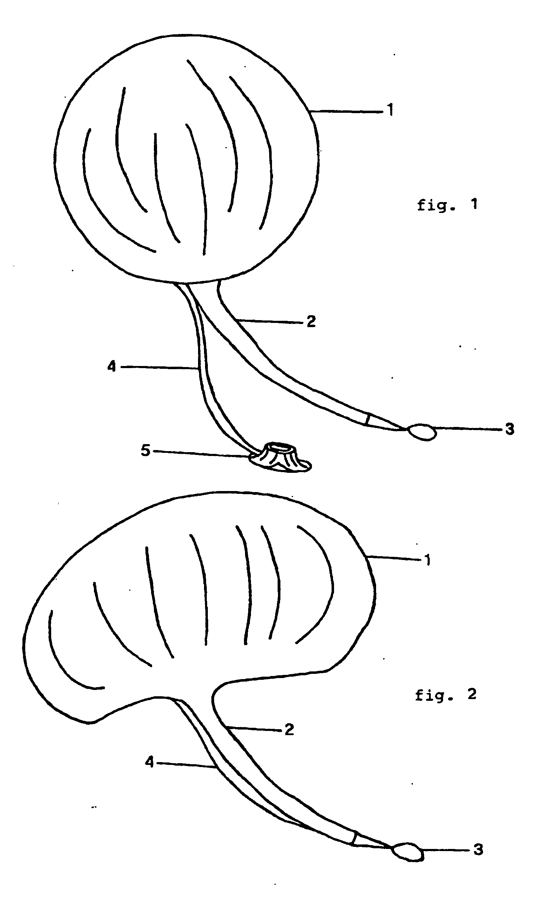

The intragastric balloon of the invention, shown in Figure 1, consists in the balloon 1, preferably made of distensible elastic silicone having a capacity between 200 to 500 c.c., approximately, and may have a circular form (as shown in Figure 1) or a bilobed or kidney form (shown in Figure 2). Said balloon 1 is provided with a rigid support 2, used as guideline and support thereof, consisting in a lengthened element such as a solid tube preferably made of silicone, unitary fixed to the balloon 1 and having a diameter such that it is larger at the end joined to the balloon 1 and it becomes smaller towards the opposite end where it is located a handle 3 to hold in place. Likewise, such rigid support 2 has a groove (not shown) extended through its length from the end attached to the balloon 1 up to approximately 15 cm ahead, disposed to receive the inflating catheter 4, which is attached to support 2 and may be easily detached from it. Said inflating catheter 4 has a diameter of approximately 1.5 to 2.0 mm and a length of approximately 20 cm.

Likewise, the invention contemplates providing an inflating valve 5 which is a rigid plastic or silicone safety valve having a 2 cm diameter and 1 cm height, approximately, which is easily available in the market and has two or three fixing flanges. Said inflating valve 5 is joined to the inflating catheter and fixed to the aponeurosis.

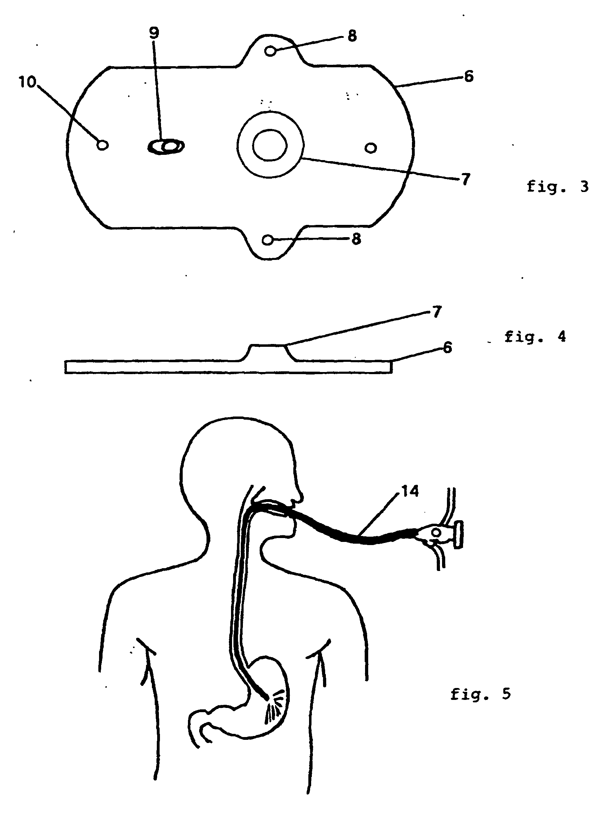

In addition, the balloon assembly of the invention, includes one fixing plate 6, shown in detail in Figure 3, which is a plate of approximately 1 to 1.5 mm wide and is preferably made of high hardness inert silicone or plastic , shown in a rectangle shape with rounded corners, but this may be different, either circular, ovoid, etc. and its diameter is about 4 to 5 cm., approximately. Said fixing plate 6, has a first hole 7 having a circular flange 8, which is adjusted and fixed to the rigid support 2 and a second hole 9 to pass the inflating catheter 4 there through, and also including several fixing holes 10, preferably being disposed 4 of those holes.

Likewise, a trocar 11 is used to make a puncture on the abdominal and gastric wall, in order to form a light for the traction, with the help of a suture through the abdominal wall, of the rigid support 2 with the catheter 4 of the balloon assembly of the invention.

In order to make clear the way the elements of the intragastric balloon assembly of the invention work, as well as its advantages, the surgical technique for its implantation is described as follows:

The balloon implanting must be made at the operating room under conventional asepsis and antisepsis measures as any surgical procedure. The anesthesia must be made under anesthesiologist control and surveillance using either general anesthesia or only sedation and a local pain-reliever in the incision zone.

Likewise, an endoscopist must be present to start the procedure and to make a permanent surveillance of the balloon placing and inflating.

The patient must be in a ventral decubitus position. Antisepsis of the abdomen is carried out and sterile fields are placed limiting the upper hemiabdomen. Local anesthesia is applied using diluted xylocaine without epinephrine on the puncture place. This site will be located after the step described below, which starts the procedure.

As shown in Figure 5, a flexible endoscope 14, preferably a central view endoscope, is introduced through the mouth and the stomach is identified by air inflation thereof, then the bottom of the gastric body is pressured with the endoscope against the major curvature, to identify the puncture site by locating the light through the abdominal wall.

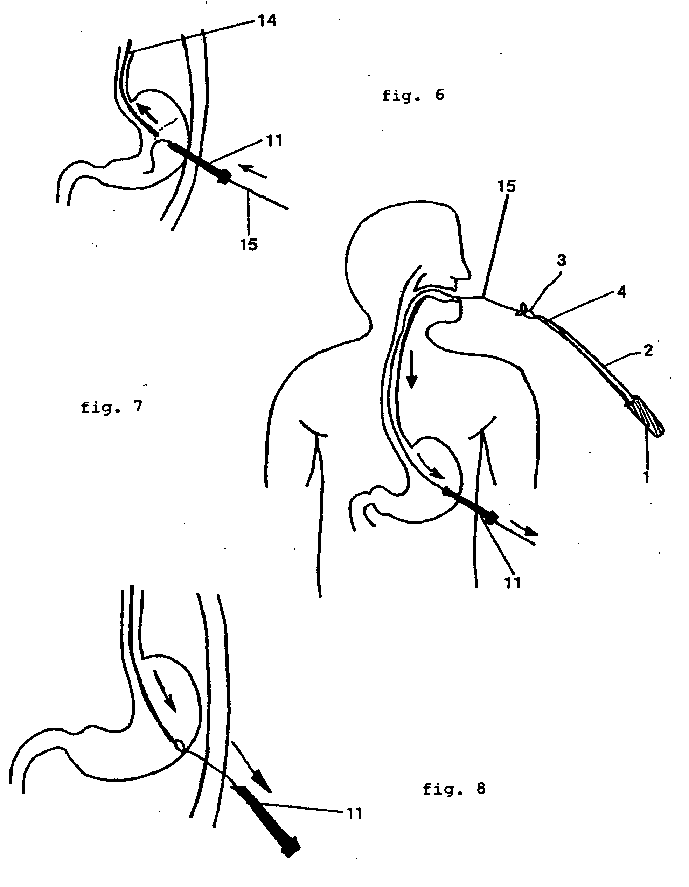

Once the puncture site has been identified, a small incision is made with a 2 mm knife, introducing there through a puncture trocar 11, as shown in Figure 6, piercing all the layers of the abdominal wall up to puncturing the stomach and watching this operation through the endoscope 14.

Upon placing the trocar 11 inside the stomach on the place where the balloon 1 is to be placed and fixed, the pointed mandrin is removed and a resistant suture 15 either prolene or mersilene , number zero or 1, is introduced through trocar 11, and it is taken by the endoscopist with a clamp and then the endoscope and the suture are removed through the mouth.

Once the suture has been removed through the mouth, the suture is tied to the thin end of the rigid support 2 of the balloon assembly, over the handle 3, placed for such purpose, as shown in Figure 7

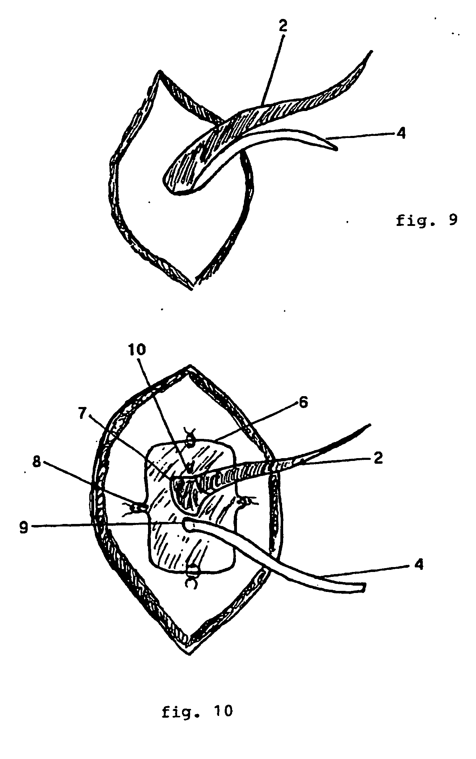

By making traction of suture 15 through the abdominal wall and with endoscopist assistance, the balloon assembly 1 is inserted along with the rigid support 2 which has the inflating catheter 4 attached up to the stomach, and then the percutaneous trocar 11 is removed and by making a firm traction of the rigid support 2 of balloon 1, it is placed in an appropriate position into the stomach (see Figure 8).

At this moment, a 5-7 cm surgical incision is carried out, preferably in a vertical or horizontal way, at the place where the rigid support 2 of the balloon 1 was removed, by such incision the skin and the cellular tissue are cut up to the aponeurosis, dissecting the aponeurosis surface, enabling it to place the fixing plate at this site. Later, the inflating catheter 4 is separated from the rigid support 2 and the solid tip thereof is cut to start inflation (see Figure 9).

After balloon 1 has been inflated with air, it may be mixed with physiologic serum, then, traction of rigid support 2 is made for the balloon 1 is perfectly attached to the gastric wall which in turns is attached to the abdominal wall at the peritoneal layer, avoiding this way any possibility of a gastric content leakage towards abdominal cavity.

Further, fixing plate 6 is placed, as shown in Figure 10, by inserting rigid support 2 into the major hole 7, having an approximate 5 mm diameter, said base having a 3-4 mm height flange, the rigid support 2 being stitched thereon, said suture may be a 1 or 2 zeros prolene, and by using this kind of sutures the fixing plate 6 may be also fixed to the abdomen anterior aponeurosis at the holes 10 stitches. The inflating catheter is introduced through the adjoining hole 9, this catheter is placed without fixation, as sustained traction is made by the rigid support 2, which is compact and soft, enabling to perforate it and tie it, by stitching it on the flange of fixing plate 6.

The rest of rigid support 2 is cut with a knife or scissors, close to the level of the flange of fixing plate 6, once it has been stitched.

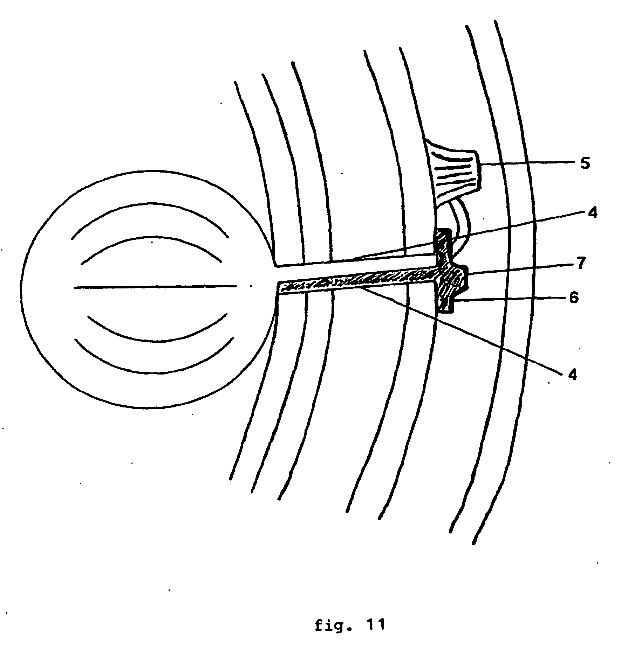

The inflating catheter 4 is cut in such a way that it is placed on the inflating valve 5, which is placed next to said incision, perforating the cellular tissue at a distance of 5-6 cm from outside and it is set in place, if necessary, by making other small incision only to place and fix the valve to the aponeurosis using stitches or staples, all the elements being arranged as shown in Figure 11.

To finish the surgery, the subcutaneous cellular tissue and the skin are closed and the endoscope is removed, verifying the correct position and inflating of the balloon inside the stomach.

It will be evident for the skilled in the art that many modifications or variations may be made to the invention herein described without separating from spirit and scope of the same, therefore, it will be understood that what has been described and shown is merely illustrative of the invention and not restrictive thereto.

## Claims

1. An intragastric balloon assembly of the kind used to reduce the food intake of persons with overweight, comprising
a) a balloon (1) made of an acid resistant distensible material;
b) a support (2) consisting in a lengthened element such as a tube, being used as a guideline and tractor to introduce the balloon (1) into the stomach through the mouth; and
c) an inflating catheter (4) joined by one end to the balloon (1) to be carried jointly with the support (2) during the procedure of introducing the assembly into the stomach;
**characterized in that**
d) the support (2) is rigid, unitarily fixed to the balloon (1) and having a diameter that is larger at the end attached to the balloon (1) and is reduced towards the opposite end, said rigid support (2) being used as a support to fix or anchor the balloon (1) in a position practically fixed, said rigid support (2) having a slot extended along all its length from the end attached to the balloon up to approximately 15 cm ahead;
e) a handle (3) is disposed at the end of the rigid support (2) opposite to the attachment to the balloon;
f) said catheter (4) is received along its length within the slot of the rigid support (2), and said catheter (4) being easily detachable from the rigid support (2);
g) the intragastric balloon assembly further comprises an inflating valve (5) to appropriately inflate or deflate the balloon that is joined to the inflating catheter and has two or three flanges for its fixation;
h) the intragastric balloon assembly further comprises a fixing plate (6) having a first hole (7) with a circular flange (8) which adjusts and fixes the rigid support (2), a second hole (9), through which the inflating catheter is introduced and several fixation holes (10) to join plate (6) by stitches to the abdominal tissue.

2. An intragastric balloon assembly according to claim 1, **characterized in that** a balloon capacity is about 200 to 500 cm³.

3. An intragastric balloon assembly according to claim 1 or 2, **characterized in that** the balloon (1) has a generally circular form.

4. An intragastric balloon assembly according to claim 1 or 2, **characterized in that** the balloon (1) has a generally bilobated form.

5. An intragastric balloon assembly according to one of claims 1 to 3, **characterized in that** the rigid support (2) is made of silicone.

6. An intragastric balloon assembly according to one of claims 1 to 5, **characterized in that** the inflating catheter (4) has a diameter of about 1.5 to 2.0 mm and a length of approximately 20 cm.

7. An intragastric balloon assembly according to one of claims 1 to 6, **characterized in that** the fixing plate (6) has an approximate thickness of 1.5 mm, and preferably is made of silicone or inert plastic of higher hardness.

8. An intragastric balloon assembly according to one of claims 1 to 7, **characterized in that** the fixing plate (6) has a generally ovoid form.

## Patentansprüche

1. Intragastrische Ballonanordnung der zur Reduzierung der Nahrungsaufnahme übergewichtiger Personen verwendeten Art, umfassend:
a) einen Ballon (1) aus einem säurefesten aufdehnbaren Material;
b) eine Stütze (2) aus einem länglichen Element, wie z.B. einem Rohr, das als Führung und Zugelement zur Einführung des Ballons (1) in den Magen durch den Mund verwendet wird; und
c) einen Aufblaskatheter (4), der mit einem Ende mit dem Ballon (1) verbunden ist, um während des Vorgangs des Einführens der Anordnung in den Magen gemeinsam mit der Stütze (2) getragen zu werden;
**dadurch gekennzeichnet, dass**
d) die Stütze (2) starr ist und gleichförmig am Ballon (1) befestigt ist und einen Durchmesser aufweist, der an dem am Ballon (1) befestigten Ende größer ist und zum gegenüberliegenden Ende hin verkleinert ist, wobei die starre Stütze (2) als Stütze zur Fixierung oder Verankerung des Ballons (1) in einer praktisch festgelegten Position dient, wobei die starre Stütze (2) einen Schlitz aufweist, der sich über ihre gesamte Länge von dem am Ballon befestigten Ende bis zu ca. 15 cm davor erstreckt;
e) ein Handgriff (3) an dem dem Befestigungspunkt des Ballons gegenüberliegenden Ende der starren Stütze (2) angeordnet ist;
f) der Katheter (4) über seine gesamte Länge im Schlitz der starren Stütze (2) aufgenommen ist, wobei der Katheter (4) sich leicht von der starren Stütze (2) lösen lässt;
g) die intragastrische Ballonanordnung ferner ein Aufblasventil (5) zum entsprechenden Aufblasen bzw. Entleeren des Ballons, das mit dem Aufblaskatheter verbunden ist und zwei oder drei Flansche zur Fixierung aufweist, umfasst;
h) die intragastrische Ballonanordnung ferner eine Befestigungsplatte (6) mit einem ersten Loch (7) mit einem kreisförmigen Flansch (8) aufweist, der die starre Stütze (2) anpasst und fixiert, einem zweiten Loch (9), durch das der Aufblaskatheter eingeführt wird, und mehreren Befestigungslöchern (10) zum Verbinden der Platte (6) mit Stichen am Bauchgewebe.

2. Intragastrische Ballonanordnung nach Anspruch 1, **dadurch gekennzeichnet, dass** das Fassungsvermögen des Ballons ca. 200 bis 500 cm³ beträgt.

3. Intragastrische Ballonanordnung nach Anspruch 1 oder 2, **dadurch gekennzeichnet, dass** der Ballon (1) eine allgemein kreisförmige Gestalt aufweist.

4. Intragastrische Ballonanordnung nach Anspruch 1 oder 2, **dadurch gekennzeichnet, dass** der Ballon (1) eine allgemein zweilappige Form aufweist.

5. Intragastrische Ballonanordnung nach einem der Ansprüche 1 bis 3, **dadurch gekennzeichnet, dass** die starre Stütze (2) aus Silikon besteht.

6. Intragastrische Ballonanordnung nach einem der Ansprüche 1 bis 5, **dadurch gekennzeichnet, dass** der Aufblaskatheter (4) einen Durchmesser von ca. 1,5 bis 2,0 mm und eine Länge von ca. 20 cm aufweist.

7. Intragastrische Ballonanordnung nach einem der Ansprüche 1 bis 6, **dadurch gekennzeichnet, dass** die Befestigungsplatte (6) eine Dicke von ca. 1,5 mm aufweist und vorzugsweise aus Silikon oder einem inerten Kunststoff höherer Härte besteht.

8. Intragastrische Ballonanordnung nach einem der Ansprüche 1 bis 7, **dadurch gekennzeichnet, dass** die Befestigungsplatte (6) eine allgemein eiförmige Gestalt aufweist.

## Revendications

1. Ensemble à ballon intragastrique du type utilisé pour réduire la prise d'aliments de personnes à surcharge pondérale, comprenant :
a) un ballon (1) réalisé en un matériau apte à être détendu, résistant à l'acide ;
b) un support (2) comprenant un élément allongé comme un tube utilisé comme une voie de guidage et d'amenée pour introduire le ballon (1) dans l'estomac par l'intermédiaire de la bouche ; et
c) un cathéter de gonflage (4) relié par une extrémité au ballon (1) destiné à être supporté conjointement avec le support (2) pendant le processus d'introduction de l'ensemble dans l'estomac ;
**caractérisé en ce que**
d) le support (2) est rigide, fixé de façon unitaire au ballon (1) et présente un diamètre qui est plus grand à l'extrémité fixée au ballon (1) et est réduit vers l'extrémité opposée, ledit support rigide (2) étant utilisé comme un support pour fixer ou ancrer le ballon (1) dans une position pratiquement fixe, ledit support rigide (2) ayant une fente prolongée sur toute sa longueur à partir de l'extrémité fixée au ballon jusqu'à environ 15 cm en avant ;
e) une poignée (3) est disposée à l'extrémité du support rigide (2) opposée à la fixation au ballon ;
f) ledit cathéter (4) est reçu sur sa longueur avec la fente du support rigide (2) et ledit cathéter (4) est facilement détachable du support rigide (2) ;
g) l'ensemble à ballon intragastrique comprend, en outre, un clapet de gonflage (5) pour gonfler ou dégonfler de façon appropriée le ballon qui est réuni au cathéter de gonflage et a deux ou trois rebords pour sa fixation ;
h) l'ensemble à ballon intragastrique comprend, en outre, une plaque de fixation (6) ayant un premier trou (7) avec un rebord circulaire (8) qui ajuste et fixe le support rigide (2), un deuxième trou (9) par l'intermédiaire duquel le cathéter de gonflage est introduit et plusieurs trous de fixation (10) pour réunir la plaque (6) par des piqûres au tissu abdominal.

2. Ensemble à ballon intragastrique selon la revendication 1, **caractérisé en ce que** la capacité du ballon est d'environ 200 à 500 cm³.

3. Ensemble à ballon intragastrique selon la revendication 1 ou 2, **caractérisé en ce que** le ballon (1) présente une forme généralement circulaire.

4. Ensemble à ballon intragastrique selon la revendication 1 ou 2, **caractérisé en ce que** le ballon (1) présente une forme généralement à deux lobes.

5. Ensemble à ballon intragastrique selon l'une quelconque des revendications 1 à 3, **caractérisé en ce que** le support rigide (2) est constitué de silicone.

6. Ensemble à ballon intragastrique selon l'une des revendications 1 à 5, **caractérisé en ce que** le cathéter de gonflage (4) présente un diamètre d'environ 1,5 à 2,0 mm et une longueur d'environ 20 cm.

7. Ensemble à ballon intragastrique selon l'une des revendications 1 à 6, **caractérisé en ce que** la plaque de fixation (6) présente une épaisseur approximative de 1,5 mm et est, de préférence, constituée de silicone ou d'une matière plastique inerte de dureté supérieure.

8. Ensemble à ballon intragastrique selon l'une des revendications 1 à 7, **caractérisé en ce que** la plaque de fixation (6) présente une forme généralement ovoide.
